# EUROPEAN PATENT SPECIFICATION

(11) **EP 0 356 603 B1**
(45) Date of publication and mention of the grant of the patent: **18.11.1993**
(21) Application number: 88850292.9
(22) Date of filing: 02.09.1988
(51) Int. Cl.: A61B 5/04

(54) **Method for ambulatory recording and ambulatory recorder**
Verfahren und Gerät zum ambulanten Registrieren
Méthode et appareil d'enregistrement ambulatoire

(43) Date of publication of application: 07.03.1990
(73) Proprietor: SYNECTICS MEDICAL AB, S-116 31 Stockholm (SE)
(72) Inventor: Essen-Möller, Anders, S-113 34 Stockholm (SE)
(74) Representative: Örtenblad, Bertil Tore

(56) References cited:
- EP-A- 0 212 278
- US-A- 4 102 332
- WESCON TECHNICAL PAPERS, vol. 20, 1976, pages 1-6, North-Hollywood, US; T. CONWAY et al.: "Microprocessor systems for blood pressure and ECG monitoring of ambulatory subjects"
- BIOMEDIZINISCHE TECHNIK, vol. 30, no. 4, April 1985, pages 76-84, Berlin, DE; R.V. KIPARSKI et al.: "Möglichkeiten der drahtlosen Biosignalerfassung mittels digitaler Speicherung"

## Description

The present invention relates to a method for ambulatory recording, for medical and especially for diagnostic purposes, by means of a portable recorder, including providing the recorder with external measurement signals.

The invention also relates to an ambulatory recorder for carrying out the method.

Ambulatory recording and recorders are previously known. Ambulatory recording makes it possible to send patients home or let them be ambulant in a hospital setting while recording physiological data. The patient carries a lightweight recorder in which the signals are stored.

Several different ambulatory recorders exist. Most common are recorders for ECG (Electrocardiogram). Other record EEG (Electroencephalogram) or pH and pressure (Motility) in the gastrointestinal tract. All recorders use specialized electronics and software which make the recorders dedicated for a special purpose for instance, gastroenterological measurements. Thus all patients are recorded in a similar manner irregardably of the actual symptoms of the patient.

With an arrangement of this kind, hospitals and other organizations or persons related to health care may have to use several different kinds of ambulatory recorders. Drug companies, for instance, often purchase ECG recorders for evaluation of antiarrhytmic drugs, blood pressure recorders for evaluation of hypertonic drugs and pH recorders for evaluation of H₂-blocking drugs. Doctors not affording or willing to learn, several types of ambulatory recorders may not supply a patient with the optimal diagnostic procedure when the patient's symptoms indicate another diagnostic ambulatory procedure than the available ambulatory recorder happens to be designed for.

Biomedizinische Technik, vol.30, no.4, April 1985, pages 76-84 shows a recorder, which may be connected to different external devices to measure differnt parameters as, for example, blood pressure, heart rate, electromyogram, temperature etc. However, all the programs which enable its microprocessor to drive said external devices, are simultaneously resident in an EPROM. The disadvantage of this system lies in the fact that said programs need a lot of memory space and that a program may not be changed, removed or added easily.

The present invention offers a solution to the above identified problems. Thus, according to the invention it is possible to use the same recorder for various diagnostic purposes. The invention contributes to diagnostic accuracy, improves patient care and reduces costs as well as learning and handling time.

Thus, the present invention relates to a method for ambulatory recording for medical and especially for diagnostic purposes by means of a portable recorder, including providing the recorder with external measurement signals.

The method is characterized in that different diagnostic procedures, tailored to the symptomatic picture of the patient, are downloaded by means of an external device, which is connected to said recorder, thereby providing said recorder with instructions of what is relevant to investigate in the particular patient.

The invention also relates to an ambulatory recorder, for medical and especially for diagnostic purposes, arranged to be provided with external measurement signals.

The recorder is characterized in that the recorder comprises a computer arranged to receive different computer programs for different measurement procedures, corresponding to different measurement purposes and measurement situations, said computer programs being downloaded from an external device, said recorder being arranged to store and execute such programs, said measurement procedures provided being tailored to the symptomatic picture of the patient.

Below, the invention is described more in detail in the form of preferred embodiments and in relation to the following drawings, where
- fig. 1 schematically shows a principal block diagram over a first embodiment of a recorder according to the invention,
- fig. 2 shows a perspective view over said first embodiment of a recorder according to the invention, and
- fig. 3 shows the control keys of the recorder according to fig. 2.

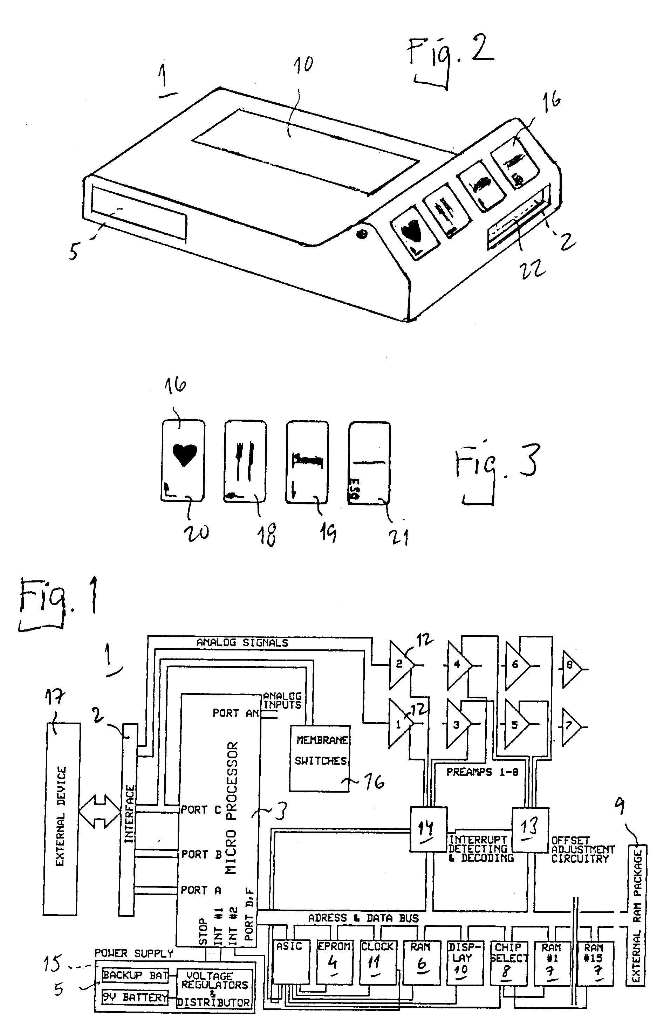

In the principal block diagram of fig. 1 1 denotes the ambulatory recorder unit. The recorder, which according to one embodiment has eight channels, comprises an interface 2 for external communication, which interface, according to one embodiment consists of only one universal plug, a 37 pin D-sub. All data, both digital and analog, pass through this unit.

The configuration according to this embodiment is as follows:
- 8-pins: for two-way transfer of digital data between a standard personal computer and the recorder, using the A port of the microprocessor 3 of the recorder. The microprocessor is described later.
- 6-pins: for controlling transfer and determine communication direction, using the B port of the microprocessor 3.
- 3-pins: for serial communication. In a synchronous mode, speeds up to 9.600 Baud is possible. Synchronous for higher speeds, using the C port of the microprocessor.
- 1-pin: for resetting of microprocessor and address registers.
- 19-pins: for analog sensor part. Every sensor input is prepared for differential signals, except for channel 7 and 8 that are buffer stages only.

There are also pins for + 5V and - 5V excitation as well as ground.

It is possible to use the recorder together with opto couplers and then connect it to e.g. a standard personal computer for on line measurements or to a telephone modem. As a result it is possible to transfer data through the modem and to shift configuration program in the ambulatory recorder.

The computer of the recorder is, according to the disclosed embodiment, a microprocessor (µP), a CMOS 78C10 from NEC. It has five digital ports, A-E, where all are used in this application, and one which takes care of analog signals (AN). The following is the use of the ports A-E and AN.
- Port A: Parallel data
- Port B: 6 pins for controlling parallel data and 2 pins for controlling (register, select) and (write/read) in a Liquid Crystal Display (LCD) module.
- Port C: 3 pins for serial data and control. 4 pins directly connected to a membrane switch panel. 1 pin is used for detecting interrupts from sensor amplifiers.
- Port D+E: Port D is used as an internal 8 bit data bus. However, it is also used as an 8 bit address bus with addresses (0-7) alternatively. Port E is used for the addresses 8-15 solely. Port D+E are connected to the blocks that can be seen in the block diagram in fig. 1,
- Port AN: Direct input for 8 analog signals. The signals are then converted to digital signals by a built-in AD converter. The analog inputs are sampled and multiplexed four by four.

The microprocessor 3 is given the necessary initialization program from an EPROM 4. This component enables the microprocessor to accept a configuration program downloaded from an external device, such as a standard personal computer. When the configuration is once loaded in a special RAM package 6, the EPROM is set in idle mode.

The microprocessor also has a stop mode feature. When operation voltage sinks below 5V, the microprocessor shuts down, saving all data in the registers.

A power supply 5 is provided and, according to the disclosed embodiment, consists of two batteries, a 9V cell for supplying all circuits in the recorder and a 3.2V lithium cell for back-up purposes.

As long as the 9V battery is good enough (> 5V), it will feed all recorder circuits and the 3.2V lithium cell is then disconnected to save life time. When the voltage goes beneath 5V, the microprocessor will be put in stop mode saving all data. The lithium cell will then take over supplying all memories (RAMs) and the clock circuit with current. The current consumption is then considerably reduced. The total power consumption is approximately 15-20mA.

The recorder according to the disclosed embodiment has one permanent memory, the EPROM capsule 4, with 8 kbyte memory. It contains all information to execute the microprocessor in order to enable a download of a configuration program from a standard personal computer. The downloaded program is put in a special 32 kbyte RAM 6 where 24 kbyte are reserved for this purpose. The remaining 8 kbyte are used for stack pointer and other necessary registers. When a program is downloaded in the RAM 6 the EPROM is automatically disconnected.

There are 15 capsules of 32 kbyte static RAM 7 in the recorder making a total of 512 kbyte memory space.

There is a special Memory Chip Select circuitry 8 controlling which of the RAM capsules that are active. The memory is mapped into banks where 1 bank equals 32 kbyte memory. Every memory capsule has the same address seen from the micro processor, but the Memory Chip Select activates one capsule at a time.

The total memory capacity can be expanded with up to 8 Mbyte by attaching a separate memory box 9 to the recorder gable. The external memory is then directly connected to the internal D and E bus.

A display 10 is provided, which is a liquid crystal type in order to keep down power consumption. It comprises two rows with room for sixteen signs each. Eight custom designed can be programmed. It has eighty internal registers. The display is controlled from the B port of the microprocessor.

A clock module 11 is provided, which is a real time type. It has an internal fifty byte register, in which different kinds of data are stored when the recorder is shut down in idle mode and run with the 3.2V battery. The clock counts in years, months, days, 12 or 24 hours, minutes and seconds. It has an interrupt output communicating with one of the interrupt inputs of the micro processor.The clock is so arranged that every time the clock changes its value an interrupt signal is sent to the microprocessor which then updates the time in its registers. Also sampling rate is controlled by the clock module.

There are six + two analog amplifiers 12 in the recorder according to the disclosed embodiment. Six amplifiers are exchangeable modules and mounted in sockets. Another two amplifiers have the amplification 1 and should be used as impedance buffers only for signal levels up to 5V. The buffers are nonexchangeable.

There are different types of amplifiers that can be plugged into the six sockets, i.e. pH, a universal amplifier called VOLT, pressure, temperature, ECG, EMG and swallow.. The differences between the amplifiers lie in the gain, frequency response, impedance and whether the input is differential or not.

The gain and offset characteristics can easily be adjusted for each of the six amplifiers by trimming corresponding potentiometers which are provided and preferably hidden under the keyboard of the recorder. Further, the recorder is so arranged that the offset factor can be set by the software on four of the six amplifiers using an offset adjustment circuitry 13 and D/A converters. This fact means that offset automatically can be set correctly by the microprocessor. Such automatic setting may also be obtained by means of an external device 17.

Four of the six amplifier sockets are also prepared for interrupt handling. There are therefore, two amplifier sockets with both offset and interrupt facilities.

There is a special unit 14 and network for detecting interrupt signals and decoding. The decoded information is intended to be sent to the microprocessor. It will then know what amplifier module that caused the interrupt. The interrupt is triggered on the negative slope of the signal.

The recorder according to the disclosed embodiment has only five external switches:
- Power on/off 15: When this is turned on the microprocessor is reset and the program executable counter provided is put at the beginning of the program.
When turned off the recorder is put in idle mode. If there is a good 9V battery in the battery compartment, the current is drawn from this. Otherwise the power is taken from the 3.2V cell. The current consumption is then low.
- Membrane 16: There are also four membrane switches 16, which are directly connected to the microprocessor's C port. They are used for patient manual event marking (four types) and to handle the downloaded configuration program. The keys are also used for setting the internal clock.

According to one embodiment, as shown in fig. 2, the recorder is provided with a set of four control keys 18, 19 20, 21, e.g. membrane switches, arranged to be used in combination with a two-dimensional screen, such as the display 10, and allow access to the information, computer programs etc., with which the recorder is provided by means of the external device and which is present in the form of items arranged in so called menues, the keys preferably being arranged so that two of the control keys 18,19 are arranged for movement to any item of said information and two of the control keys 20, 21 are arranged for confirming a chosen item and for moving back to the previous menue, respectively. The described use is relevant in a computer mode of a dual mode set of the recorder. In the other mode, recording mode, the keys are used e.g. for marking specific events or the like.

In fig. 3 the keys 18,19,20,21 of the recorder in fig.1 and 2 are specified. Two keys 18,19 are for obtaining menues and items on a menue displayed on the screen. The items may be different measurement programs etc. One key 20 is used for confirming items. Confirmation may correspond to a choice of a certain program. The remaining key 21 is used to move back to the previous menue.

In fig. 1 17 denotes an external device, such as a so called personal computer, from which data programs for the recorder is intended to be transferred to the recorder via the interface 2. The external device does not have to be a computer but several different kinds of devices may be used, e.g. a tape recorder, a telephone etc.

Thus, the recorder is arranged to measure analog signals and store the result, signals being provided via the interface. Of course the recorder may be arranged to process measured signals, e.g. by means of the computer.

Further, it is arranged to export measured signals via the interface to an external device, e.g. for real time displaying on an external device.

According to one embodiment the recorder is arranged to modify or exchange the program of the computer depending on measured signals. Thus, e.g. when signals exceeding a pre-determined value are measured, the recorder is arranged to modify or exchange the program of the computer, e.g. so that other parameters are measured or so that certain actions, such as the start of an infusion pump, are initiated. In this case the recorder is arranged to process measured signals.

According to a preferred embodiment there is only one single port 22 for communication with external devices and signal sources, one at a time. In this way, when the recorder is connected to the external device 17 it cannot be connected to the patient or the corresponding. According to another embodiment the measured subject, the patient, is electrically isolated from the external device, yet only one communication port is provided, although the recorder can be connected to the external device and the signal sources at the same time.

The method according to the invention and the way an ambulatory recorder according to the invention works may be described in the following way.

Programs corresponding to different purposes, such as different diagnostic situations or measurement situations, are prepared, using e.g. a personal computer, this making it very simple to amend the programs in order to tailor it for the purpose, e.g. for a certain patient. The programs are transferred to the recorder via the interface, the recorder being connected to the external device 17, the P.C. or some other device, and disconnected from the measuring subject, the patient.

When applicable the proper diagnostic procedure is chosen and downloaded into the recorder, the computer mode being used. Physiological input signals etc can then be fed into the recorder from the patient or the corresponding and can be transferred for storage or real time display to a computer.

Above the invention has been described in detail and as is obvious the invention offers important advantages compared to what is previously known. Thus, the invention offers an extremely flexible tool when it comes to perform measurements for diagnostic purposes and even therapeutical purposes. The invention makes it possible to obtain exactly the right measurement procedure for each diagnostic measurement situation.

Further, there are of course many different kinds of measurement procedures for which the invention can be used. Primarily diagnostic procedures are aimed at but it is possible to adapt the recorder for therapeutical purposes.

Regarding the control keys, of course the number and functions of the keys may be varied. The number may be greater or less than four adding or cancelling special features and different possibilities to control the configuration program.

## Claims

1. Method for ambulatory recording, for medical and especially for diagnostic purposes by means of a portable recorder, including providing the recorder with external measurement signals, **characterized in** that different diagnostic procedures, tailored to the symptomatic picture of the patient, are downloaded by an external device (17), which is connected to said recorder, therby providing said recorder (1) with instructions of what is relevant to investigate in the particular patient.

2. Method according to claim 1, further **characterized in** that, in the case of analog external signals being provided, such signals are measured and, when applicable, processed by means of the computer.

3. Method according to claim 1 or 2, further **characterized in** that measured signals are exported from the recorder for real time displaying on an external device.

4. Method according to claim 1, 2 or 3, further **characterized in** that depending on measured signals the instructions of the recorder are modified or exchanged from an external device.

5. Method according to claim 1, 2, 3 or 4, further **characterized in** that access to the information, with which the recorder is provided by means of the external device, and which is present in the form of items arranged in so called menues, is obtained by means of a set of four control keys (18,19,20,21), the control keys (18,19,20,21) being used in combination with a two-dimensional screen, two of the control keys (18,19) being used for movement to any item of said information and two of the control keys (20,21) are used for confirming a chosen item and for moving back to the previous menue respectively.

6. Ambulatory recorder for medical and especially for diagnostic purposes, arranged to be provided with external measurement signals, **characterized in** that said recorder (1) comprises a computer arranged to receive different computer programs for different measurement procedures, corresponding to different measurement purposes and measurement situations, said computer programs being downloaded from an external device (17), said recorder (1) being arranged to store and execute such programs, said measurement procedures provided being tailored to the symptomatic picture of the patient.

7. Recorder according to claim 6, further **characterized in** that a set of, preferably, four control keys (18,19,20,21) is provided and arranged to be used in combination with a two-dimensional screen (10) and allow access to the information, with which the recorder (1) is provided by means of the external device and which is present in the form of items arranged in so called menues, two of the control keys (18,19) being arranged for movement to any item of said information and two of the control keys (20,21) being arranged for confirming a chosen item and for moving back to the previous menue, respectively.

8. Recorder according to claim 7, further **characterized in** that it is arranged so that one mode, a computer mode, is available for access to said information by means of the set of control keys (18,19,20,21) and another mode, a recorder mode, is available for using the control keys to add special information to the recorded measurement signals.

9. Recorder according to claim 6, 7 or 8, further **characterized in** that only one single plug (22) is provided for communication with external devices (17) and analog signal sources, the measured subject, respectively.

10. Recorder according to claim 6, 7, 8 or 9, further **characterized in,** that it is arranged so that special actions, such as the start of an infusion pump, are initiated depending on measured signals.

11. Recorder according to claim 6, 7, 8, 9 or 10, further **characterized in** that pre-amplifiers (12) provided for received analog measurement signals are arranged to be set and controlled by means of an external device (17) so that pre-amplification may be automatically adapted to the measurement situation.

## Patentansprüche

1. Verfahren zur ambulanten Aufzeichnung für medizinische und insbesondere diagnostische Zwecke mittels eines mobilen Recorders einschliesslich dessen Versorgung mit externen Mess-Signalen, dadurch gekennzeichnet, dass verschiedene der Symptomatik des Patienten angepasste Diagnoseprogramme von einem an besagten Recorder angeschlossenen externen Gerät (17) aus geladen werden, welches auf diese Weise den Recorder (1) mit Anweisungen darüber versieht, was bei der Untersuchung des betreffenden Patienten relevant ist.

2. Verfahren gemäss Anspruch 1, ferner dadurch *gekennzeichnet,* dass, falls externe Analogsignale zugeführt werden, solche Signale gemessen werden und dass sie, wenn sie anwendbar sind, mit Hilfe des Computers verarbeitet werden.

3. Verfahren gemäss Anspruch 1 oder 2, ferner dadurch *gekennzeichnet,* dass diese Mess-Signale vom Recorder zu einem externen Gerät geführt werden zwecks Realzeitanzeige.

4. Verfahren gemäss Anspruch 1, 2 oder 3, ferner dadurch *gekennzeichnet,* dass, abhängig von Mess-Signalen, die Anweisungen des Recorders von einem externen Gerät aus verändert oder ausgetauscht werden.

5. Verfahren gemäss Anspruch 1, 2, 3 oder 4, ferner dadurch *gekennzeichnet,* dass der Zugriff auf die Information, mit welcher der Recorder von einem externen Gerät aus versorgt wird und welche in Form von als sog. Menüs arrangierten Feldern vorliegt, mittels eines Satzes von vier Steuertasten (18, 19, 20, 21) erfolgt, wobei die Steuertasten (18, 19, 20, 21) in Kombination mit einem zweidimensionalen Anzeigeschirm verwendet werden, und wobei jeweils zwei der Steuertasten (18, 19) zur Positionierung auf das jeweilige Feld besagter Information und zwei Steuertasten (20, 21) zur Bestätigung eines ausgewählten Feldes sowie für den Rücksprung ins vorhergehende Menü benutzt werden.

6. Ambulanter Recorder für medizinische und insbesondere diagnostische Zwecke, welcher eingerichtet ist, um mit externen Mess-Signalen versorgt zu werden, dadurch *gekennzeichnet,* dass besagter Recorder (1) einen für die Aufnahme verschiedener Computerprogramme für verschiedene Messverfahren ausgerüsteten Computer enthält, die verschiedenen Messzwecken und -situationen entsprechen, wobei diese Computerprogramme von einem externen Gerät (17) aus ladbar sind, wobei besagter Recorder (1) zum Speichern und Ausführen solcher Programme ausgerüstet ist, und wobei besagte Messverfahren der Symptomatik des Patienten angepasst werden.

7. Recorder gemäss Anspruch 6, ferner dadurch *gekennzeichnet,* dass ein Satz von vorzugsweise vier Steuertasten (18, 19, 20, 21) für den Gebrauch in Kombination mit einer zweidimensionalen Anzeige (10) verwendet wird und den Zugriff auf die Information erlaubt, mit welcher der Recorder (1) durch ein externes Gerät versorgt wird und welche in Form von als sog. Menüs arrangierten Feldern vorliegt, wobei jeweils zwei der Steuertasten (18,19) zur Positionierung auf jedes beliebige Feld besagter Information bzw. zwei weitere Steuertasten (20, 21) zur Bestätigung eines ausgewählten Feldes sowie zum Rücksprung ins vorhergehende Menü eingerichtet sind.

8. Recorder gemäss Anspruch 7, ferner dadurch *gekennzeichnet,* dass er derart eingerichtet ist, dass ein Modus, Computermodus, für den Zugriff auf besagte Information über einen Satz von Steuertasten (18, 29, 20, 21) zur Verfügung steht und ein anderer Modus, Recordermodus, zur Verfügung steht, um die Steuertasten zum Ergänzen der aufgezeichneten Mess-Signale mit einer spezifischen Information zu verwenden.

9. Recorder gemäss Anspruch 6, 7 oder 8, ferner dadurch *gekennzeichnet,* dass zur Kommunikation mit externen Geräten (17) bzw. mit analogen Signalquellen, dem Messobjekt, ein einziger Stecker (22) vorgesehen ist.

10. Recorder gemäss Anspruch 6, 7, 8 oder 9, ferner dadurch *gekennzeichnet,* dass er derart eingerichtet ist, dass spezielle Aktionen, wie der Start einer Infusionspumpe, in Abhängigkeit von den Mess-Signalen veranlasst werden.

11. Recorder gemäss Anspruch 6, 7, 8, 9 oder 10, ferner dadurch *gekennzeichnet,* dass die Vorverstärker (12), die für den Empfang analoger Mess-Signale vorgesehen sind, zur Einstellung und Steuerung durch ein externes Gerät (17) eingerichtet sind, so dass die Vorverstärkung automatisch der Mess-Situation angepasst werden kann.

## Revendications

1. Procédé d'enregistrement ambulatoire à des fins médicales, et en particulier de diagnostics, au moyen d'un enregistreur portable, consistant à fournir à l'enregistreur des signaux de mesure extérieurs, caractérisé en ce que des processus de diagnostic différents, qui sont façonnés d'après l'image symptomatique du malade, sont déchargés par un dispositif extérieur (17), qui est connecté audit enregistreur, fournissant ainsi audit enregistreur (1) des instructions de ce qui est pertinent pour examiner le malade particulier.

2. Procédé suivant la revendication 1, caractérisé en outre en ce que, dans le cas où des signaux analogiques extérieurs sont prévus, de tels signaux sont mesurés et, lorsqu'ils sont appliqués, sont traités au moyen de l'ordinateur.

3. Procédé suivant la revendication 1 ou 2, caractérisé en outre en ce que des signaux mesurés sont délivrés par l'enregistreur en temps réel sur un dispositif d'affichage extérieur.

4. Procédé suivant la revendication 1, 2 ou 3, caractérisé en outre en ce que des instructions de l'enregistreur sont modifiées ou échangées à partir d'un dispositif extérieur, en fonction des signaux mesures.

5. Procédé suivant la revendication 1, 2, 3 ou 4, caractérisé en outre en ce que l'accès à l'information, dont l'enregistreur est pourvu au moyen du dispositif extérieur et qui est présente sous la forme d'éléments agencés dans ce que l'on appelle des menus, est obtenu au moyen d'un ensemble de quatre clés de contrôle (18,19,20,21), les clés de contrôle (18,19,20,21) étant utilisées en combinaison avec un écran à deux dimensions, deux des clés de contrôle (18,19) étant utilisées pour un mouvement vers un élément quelconque de ladite information et deux des clés de contrôle (20,21) étant utilisées pour confirmer l'élément choisi et pour revenir au menu précédent respectivement.

6. Enregistreur ambulatoire destiné à des fins médicales et en particulier de diagnostics agencés pour recevoir des signaux de mesures extérieurs, caractérisé en ce que ledit enregistreur (1) comprend un ordinateur, qui est agencé pour recevoir des programmes d'ordinateur différents pour des processus de mesure différents, correspondant à différents objets et situations de mesure, lesdits programmes d'ordinateur étant déchargés à partir d'un dispositif extérieur (17), ledit enregistreur (1) étant agencé pour stocker et exécuter de tels programmes, lesdits processus de mesure prévus étant façonnés d'après l'image symptomatique du malade.

7. Enregistreur suivant la revendication 6, caractérisé en outre en ce qu'un ensemble, de préférence, quatre clés de contrôle (18,19,20,21) est prévu et est agencé pour être utilisé en combinaison avec un écran à deux dimensions (10) et pour permettre l'accès à l'information dont l'enregistreur (1) est pourvu au moyen d'un dispositif extérieur et qui est présente sous la forme d'éléments agencés dans ce que l'on appelle des menus, deux desdites clés de contrôle (18,19) étant agencées pour le mouvement d'un élément quelconque de ladite information et deux des clés de contrôle (20,21) étant agencées pour confirmer l'élément choisi et pour revenir au menu précédent respectivement.

8. Enregistreur suivant la revendication 7, caractérisé en outre en ce qu'il est agencé de façon qu'un mode, mode d'ordinateur, soit disponible pour l'accès à ladite information au moyen d'un ensemble de clés de contrôle (18,19,20,21) et qu'un autre mode, un mode d'enregisteur, soit disponible pour utiliser les clés de contrôle afin d'ajouter des informations particulières aux signaux de mesure enregistrés .

9. Enregistreur suivant la revendication 6, 7 ou 8, caractérisé en outre en ce qu'une seule prise(22) est prévue pour établir une communication avec des dispositifs extérieurs (17) et des sources de signaux analogiques, et respectivement l'objet mesuré.

10. Enregistreur suivant la revendication 6, 7, 8 ou 9, caractérisé en outre en ce qu'il est agencé de façon que des actions particulières, telles que l'amorce d'une pompe à infusion, soient lancées en fonction de signaux mesurés.

11. Enregistreur suivant la revendication 6,7,8,9 ou 10, caractérisé en outre en ce que des pré-amplificateurs (12) prévus pour recevoir des signaux de mesure analogiques sont agencés pour être réglés et contrôlés au moyen d'un dispositif extérieur (17) de sorte que la pré-amplification puisse être adaptée automatiquement à la situation de mesure.
